**Europäisches Patentamt**

**European Patent Office**

**Office européen des brevets**

(11) Publication number: **0 178 108**
A2

# EUROPEAN PATENT APPLICATION

(21) Application number: **85306978.9**

(22) Date of filing: **30.09.85**

(51) Int. Cl.⁴: **A 61 F 13/18**

(30) Priority: **02.10.84 GB 8424851**

(43) Date of publication of application: **16.04.86**
**Bulletin 86/16**

(84) Designated Contracting States: **BE CH DE FR GB LI NL**

(71) Applicant: **Smith and Nephew Associated Companies p.l.c., 2, Temple Place Victoria Embankment, London WC2R 3BP (GB)**

(72) Inventor: **Lloyd, Ronald, 63 Cambridge Road, Sawbridgeworth Hertfordshire (GB)**
Inventor: **Carter, Andrew James, 7 Springhall Road, Sawbridgeworth Hertfordshire (GB)**

(74) Representative: **Cole, William Gwyn, Dr., Smith and Nephew Research Limited Gilston Park, Harlow Essex CM20 2RQ (GB)**

(54) **Hygienic absorbent pads.**

(57) A hygienic absorbent pad which comprises a liquid pervious cover layer, an absorbent layer and a liquid pervious intermediate layer between the liquid pervious cover layer and the body facing surface of the absorbent layer characterised in that said intermediate layer comprises an embossed plastics net.

# HYGIENIC ABSORBENT PADS

The invention relates to hygienic absorbent pads.

Conventional hygienic absorbent pads such as sanitary towels, diapers, incontinence pads and alike normally comprise an absorbent layer and a liquid pervious layer which covers the body facing surface of the absorbent layer. The cover layer of these pads in use makes direct contact with the user's skin. Any body fluid exudate flowing onto the pad will therefore first contact the cover layer and then be transmitted therethrough to the absorbent layer. Conventional hygienic absorbent pads suffer from the problem that in use, pressure exerted on the pad can cause absorbed body fluid to be squeezed out of the absorbent layer and to be returned back to the surface of the pad to wet the cover layer. This fluid wet-back characteristic of conventional absorbent pads is undesirable because it provides the pad with a wet surface which may make the pad uncomfortable to wear and may also result in the wearer's skin being in prolonged contact with fluid exudate such as urine which can cause skin irritancy and rashes. It is known from British Patent No. 1,160,625 to provide hygienic absorbent pads with an intermediate layer of wadding made of viscose rayon or polypropylene fibres which is positioned between the cover and absorbent layers of the pad. This intermediate layer separates and therefore prevents contact between these two layers, thereby inhibiting fluid wet back. Tissue waddings made of viscose rayon fibres or polypropylene fibres however have not been found entirely satisfactory for use as an intermediate layer. Viscose rayon fibre wadding is highly absorbent. Contact between the cover layer and an intermediate layer of such a wadding, containing absorbed body fluid exudate, may therefore maintain the cover layer in a wet state. A wadding of polypropylene fibres, because of the hydrophobic nature of the fibres, does not normally readily allow the transmission of body fluids. Any body fluid exudate contacting an intermediate layer of such wadding is therefore likely to stay on the surface of the layer and wet the cover layer before being transmitted to the absorbent layer.

Furthermore wadding layers of viscose rayon or polypropylene fibres lack resilience and are therefore likley to be compacted within an absorbent pad in use, thus reducing the separation between the cover and absorbent layers.

An absorbent pad has now been found which overcomes the problems of the prior art by employing a liquid pervious intermediate layer between the cover layer and the absorbent layer which provides a good separation between these layers and has a good resistance to compaction in use.

Accordingly the present invention provides an hygienic absorbent pad which comprises a liquid pervious cover layer, an absorbent layer, and a liquid pervious intermediate layer between the liquid pervious cover layer and the body facing surface of the absorbent layer, characterised in that said intermediate layer comprises an embossed plastics net.

The embossed plastics net employed in this invention tends to be too rough to use as a cover layer but this problem is negated in the hygienic absorbent pad of the invention by means of the liquid pervious cover layer.

The term "embossed plastics net" as used herein means a plastics net which has a pattern of raised or depressed areas or a pattern of both raised and depressed areas. The term is not intended to indicate any method of forming the raised or depressed areas.

The term "net" as used herein means a structure in which the ribs or strands and junctures are formed integrally for example during manufacture.

Embossed plastics nets with such a pattern of areas have a three dimensional character. When used as an intermediate layer in the absorbent pad of the invention such an embossed plastics net can provide good separation of the cover layer from the absorbent layer. It has been found that this inhibits fluid wet-back to the surface of the pad in use. This inhibition of wet-back has been found to be particularly effective in sanitary towels which therefore form a preferred aspect of the present invention.

The intermediate layer can comprise more than one layer of embossed plastics net, for example, two such layers which may have the same or a different pattern of areas. It is preferred, however, that the intermediate layer comprises a single layer of embossed plastics net.

The embossed plastics net used in the invention is aptly formed by providing a pattern of raised or depressed areas or both such areas in a plastics net.

Suitable plastics nets which can be used to form the embossed plastics nets used in the invention include the plastics nets described as suitable for use as cover layers on absorbent pads. Such plastics nets have good fluid transmission properties. The plastics net should preferably be made of a hydrophobic material such as a polyolefine, for example polypropylene or high density polyethylene to render the fabric relatively non-absorbent.

Favoured plastics nets for use in forming the embossed plastics nets used in the invention are those disclosed hereinafter in relation to the liquid pervious cover layer of the invention.

Suitably the plastics net used to form the embossed plastics net used in the invention has a weight per unit area of 5 to 30g/m² and preferably a weight per unit area of 7 to 12g/m².

Suitably the plastics net used to form the embossed plastics net used in the invention has a thickness of 0.040mm to 0.180mm and preferably a thickness of 0.050mm to 0.120mm.

The pattern of raised and/or depressed areas provided on the plastics net converts the normal thin planar form of the plastics net into a thicker, more obviously three dimensional form.

In order to provide good separation between the cover and absorbent layers of the absorbent, it is preferred that the embossed plastics net has a thickness of not less than 0.3mm and preferably a thickness of not less than 0.5mm (from upper notional surface to lower notional surface). The thickness of the embossed plastics net can suitably be 0.3 to 1.4mm and preferably be 0.5 to 1.2mm.

The raised or depressed areas in the embossed plastics net can have a variety of shapes. Suitable shapes include straight and curved lines (grooves or ridges), triangles, squares, rectangles, parallelograms, circles, ellipses and like shapes. The raised or depressed portions of these areas, however will normally have a curved profile.

The pattern of raised or depressed areas in the embossed plastics net will normally be a regular pattern. Such a regular pattern preferably comprises parallel rows of the raised or depressed areas or both such areas in which the areas in adjacent rows are in aligned or staggered relationship.

The raised or depressed areas should be sufficiently small to provide the embossed plastics net with good resistance to permanent compaction so as to maintain a good separation between cover and absorbent layer of the pad in use.

It is preferred therefore that the embossed plastics net has greater than 4 per cm and preferably has greater than 6 per cm raised or depressed areas in one direction.

A favoured embossed plastics net for use in the invention has an alternating pattern of parallel continuous lines of raised and depressed areas. Such an embossed plastics net thus exhibits an undulating profile of alternating ridges and troughs. It is preferred, however, that when the net is of the type, as mentioned hereinafter, which has longitudinal ribs interconnected by the fibrillated strands, the alternating ridges and troughs extend in a direction which is transverse to the longitudinal ribs of the net.

An apt embossed plastics net of this type has 10 per cm parallel ridges and troughs with a pitch of 1mm and has a thickness of 0.5 to 0.7mm.

The liquid pervious cover layer used in the invention can be any of the cover layers conventionally used for hygienic absorbent pads. Preferably the cover layer is made of hydrophobic polymer such as polyolefine, for example polypropylene, high density polyethylene or polyester. Such cover layers are less likely to retain fluid exudate at the pad surface.

Favoured cover layers for use in the invention include plastics nets and non-woven fabrics such as bonded non-woven fabrics for example spun bonded and thermally bonded non-woven fabrics.

The bonded non-woven fabric used in the invention can suitably have a weight per unit area of 2 to 40g/m² and can preferably have a weight per unit area of 10 to 30g/m².

Similarly, the bonded non-woven fabric used in the invention can suitably have a thickness of 0.025mm to 0.110mm and can preferably have a thickness of 0.040mm to 0.075mm.

Favoured bonded non-woven fabrics are spun bonded polypropylene fabrics, for example Lutravil VP 605 available from Lutravil Spinnvlies.

Suitable cover layers for use in the invention include plastics nets.

Suitable plastic nets for use as a cover layer in the invention include those disclosed in British Patent No. 1,548,865. These nets comprise straight parallel, smooth, longitudinal ribs interconnected by fibrillated strands (that is small fibre-like strands). Such nets have strength, porosity and softness properties which make them highly suitable for use as cover for a sanitary towel. Other suitable plastics nets include those disclosed in British Patent No. 1055963. Such nets comprise thick junctures interconnected by thin strands.

Suitably the plastic nets for use as a cover layer in the invention have a weight per unit area of 2 to 30g/m² and preferably a weight per unit area of 4 to 12g/m².

Suitably the plastics nets for use as a cover layer in the invention have a thickness of 0.030mm to 0.180mm and preferably a thickness of 0.04mm to 0.120mm.

Favoured plastics nets comprise a blend of a polyolefine and incompatible polymer. Particularly favoured plastic nets comprise a blend of high density polyethylene and high impact polystyrene. Apt integral nets of this type include those available as Net 909 (Trade Mark) grades A4, A7, H8C and DHM (available from Smith & Nephew Plastics Ltd.). Such apt nets have a weight per unit area of 5 to 11.5g/m² and a thickness of 0.04mm to 0.120mm.

The absorbent layer used in the invention can be any of the absorbent layers used in conventional hygienic absorbent pads. Suitable absorbent materials for such layers include comminuted/fluffed wood pulp, carded cotton webs, viscose rayon fibres, tissue wadding, grafted cellulose super-absorbents, polymeric super-absorbents or mixtures thereof. The absorbent layer can optionally contain an insert such as a tissue wadding to aid fluid distribution within the layer.

The hygienic absorbent pad of the invention has an intermediate layer of embossed plastics net between the liquid pervious cover layer and the body facing surface of the absorbent layer. The intermediate layer of embossed plastics net can cover only a portion of the body facing surface of the absorbent layer, for example the central portion of such surface. However, it is preferred that the intermediate layer of embossed plastics net covers the whole of the body facing surface of the absorbent layer.

The hygienic absorbent pad of the invention can be a diaper, an incontinence pad, a sanitary towel or like absorbent pad. However, a sanitary towel is the preferred hygienic absorbent pad of the invention, as it has been found that the use of an embossed plastic net intermediate layer in a sanitary towel of the invention is particularly suitable for inhibiting fluid wet-back to the surface of the towel in use.

Sanitary towels normally comprise an elongate absorbent layer in the form of an absorbent core. In a preferred sanitary towel of the invention, the embossed plastics net intermediate layer extends around the longitudinal side edges of the core and preferably over the non-body facing surface thereof to surround and thus form a wrapper for the core. However, it is not necessary that the whole of the plastics net wrapper is embossed to inhibit fluid wet-back. Thus in another preferred sanitary towel of the invention the intermediate layer of embossed plastics net fabric forms part of a wrapper of plastics net which surrounds the absorbent core. The embossed plastics net of such a wrapper can conveniently be the portion of the wrapper which covers the body facing surface of the core.

Absorbent pads of the invention can optionally have a fluid impermeable barrier layer such as thin plastics barrier film, for example a polyethylene film over the non-body facing surface of the absorbent layer to prevent penetration of body fluid exudate through the pad. Absorbent pads of the invention, in particular a sanitary towel, can optionally have adhesive for example in the form of strips, on its non-body facing surface to allow attachment of the pad to a supporting garment.

In absorbent pads of the invention which comprise both a liquid pervious cover layer and intermediate layer made of a hydrophobic polymer, it is preferred that the cover layer be treated with a surfactant, for example a non-ionic surfactant to promote the transmission of body fluid exudate through the layers.

The cover layer can be treated with surfactant by any suitable method such as spraying, dip coating or roller coating.

The embossed plastics net used in the invention can be formed by embossing a suitable plastics net by a conventional embossing method, using for example, embossing rollers or plates or any other convenient method which can provide a pattern of raised and/or depressed areas in the plastics net.

A preferred method of forming the embossed plastics net used in the invention comprises passing a plastics net between and in contact with two rollers, one or both of which have a pattern of raised and/or depressed areas on their surface.

The plastics net or embossing rollers or both can be heated to a suitable temperature to assist in deforming the plastics net.

In an embossing method which employs two pressure rollers, one of which is a smooth roller and the other roller has a pattern of areas on its surface, it is preferred that the smooth roller has a soft resilient surface, for example of rubber or foam, which allows the areas of the pattern on the other roller, which is normally made of hard material such as metal, to press into and permanently deform the plastics net.

A preferred method for forming the embossed plastics net of the invention, however, comprises passing a plastics net between and in contact with two rollers, which have an intermeshing pattern of raised and depressed areas on their surfaces. A favoured method employs gear type rollers having axial ridges (and grooves) of approximate truncated triangular shape which intermesh. Apt rollers of this type have 10 per cm parallel axial ridges and grooves of depth 0.7mm.

The embossed plastics net formed by such embossing methods employing two pressure rollers can conveniently be in the form of a strip which can be used to form the absorbent pad of the invention. Such a strip may have embossed areas over the whole of the strip or over a central portion thereof only. The width of the embossed area can be adapted to the width of the absorbent layer of the pad. The width of the strip can be adapted to the width of the absorbent layer or to a width which is suitable to form a wrapper around the absorbent layer for example the absorbent core of a sanitary towel.

The hygienic absorbent pad of this invention may be assembled by hand or by a conventional process of assembling such articles.

The process of preparing the hygienic absorbent pad of the invention can be a conventional process for preparing such absorbent pads in which the embossed plastics net is provided as an intermediate layer between the liquid pervious cover layer and the absorbent layer. In a continuous process such layers are normally in the form of continuous strips. In such a continuous process the strip of embossed plastics net can be fed between a strip of liquid pervious cover layer and a strip of absorbent layer.

In a continuous process of forming sanitary towels of the invention, a continuous length of absorbent core material can conveniently be fed onto a continuous strip of wholly or centrally embossed plastics net so that one face of the core is adjacent to an embossed portion of the non-woven fabric. The plastics net if of suitable width, can then be folded around the core and the overlapping portions at the back of the core sealed, for example by application of adhesive. A liquid pervious cover strip can then be applied in a similar manner to form an outer wrapper for the core.

The continuous length of wrapped core can then be cut into suitable lengths before or after the overlapping portions of the wrapper are sealed adjacent to the ends of the core, for example by heat sealing to form sanitary towels of the invention.

The invention will now be illustrated by way of example only by reference to the following drawings in which;

Figure 1 is a cross-section of a sanitary towel of the invention.

Figure 2 is a cross-section of another sanitary towel of the invention.

Figure 3 is a cross-section of a further sanitary towel of the invention.

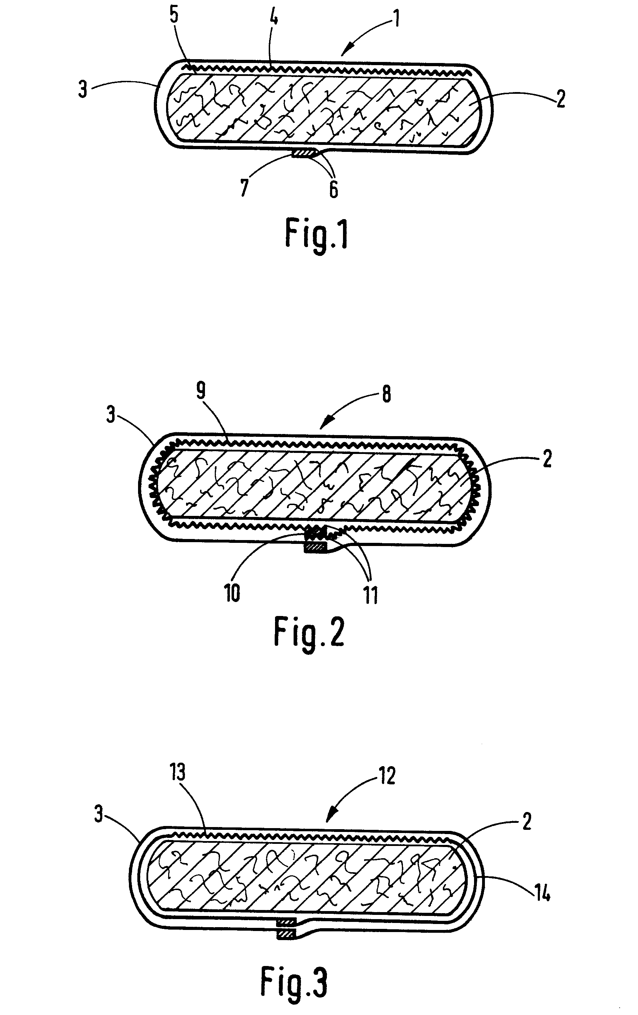

Figure 1 shows a sanitary towel (1) which comprises an absorbent core (2) and a liquid pervious cover layer (3) which surrounds the core.

Sanitary towel (1) has an intermediate layer (4) of an embossed plastics net between the cover layer (3) and the absorbent core (2) which extends over the width of the body facing surface (5) of the absorbent core (2). The cover layer (3) has overlapping edge portions (6) which are sealed by adhesive (7).

Figure 2 shows a sanitary towel (8) which is similar to that of Figure 1 in which the intermediate layer (9) of embossed plastics net extends around the absorbent core to form a wrapper and is sealed by adhesive (10) at its overlapping edge portions (11).

Figure 3 shows a sanitary towel (12) which is similar to that of Figure 2 in which the intermediate layer (13) of embossed plastics net forms part of a plastics net wrapper (14) for the absorbent core (2).

The embossed plastics net of intermediate layers 4, 9 and 13 of the sanitary towels of Figures 1 to 3 are represented diagrammatically.

The invention will now be illustrated by reference to the following examples.

Example 1

Preparation of Embossed Plastics Net

A 160mm wide strip of a plastics net having longitudinal ribs (Net 909 Ref. H8, weight per unit area of $9g/m^2$ and a thickness of 0.06mm) was passed through the nip of driven intermeshing gear rollers, heated to a temperature of 70-80°C, to form a strip having a 5cm wide inner central strip portion embossed with transverse corrugations. Both gear rollers had a pattern of 10 per cm parallel axial rib-like projections (and grooves) 0.7mm high of truncated triangular shape. The embossed portion of the strip had a thickness of 0.63mm.


Preparation of Sanitary Towel


An absorbent core (width 60mm, length 216mm) of comminuted fluff pulp (weight 6.6 grams), having central layer insert of folded tissue wadding, was placed centrally onto the strip (length 216mm) of embossed plastics net as prepared above and the edges of the strip folded around the longitudinal side edges and back face of the core and the overlapping edge portions of the strip sealed by application of adhesive. The front face of the wrapped core was then placed centrally onto a strip (length 260mm and width 180mm) of plastics net reference DHM (weight per unit area $6g/m^2$) previously treated with a 1.0% aqueous solution of a non-ionic surfactant (Amoa P231 available from Amoa Chemical Co.) which was then wrapped around the embossed plastics net wrapped core and its overlapping portions at the back face of the core and sealed by application of adhesive. The portions of the net cover overlapping the ends of the core were then heat sealed to form a sanitary towel of the invention. The sanitary towel so formed thus had an intermediate layer of embossed plastics net between the absorbent layer and liquid pervious net layer covering the body facing side of the absorbent layer.

Fluid Wet-back Test

Sanitary towels made according to Example 2 were subjected to a fluid wet-back test which was carried out in the following manner.

A 1Kg flat weight (dimensions 10 x 5 x 2cm) was placed on the towel and left for 2 mins and then removed. 5ml of 1% saline solution coloured with lissamine green dye was delivered onto the centre of the towel by means of a syringe pump at a rate of 1ml/min and at a height of 1cm above the towel surface. A preweighed stack of filter papers (Whatman No. 1 filter papers) were then placed over the wetted area of the towel and a 1Kg flat weight (dimensions 10 x 5 x 2cm) placed on the filter papers. After 1 minute, the filter papers were removed and weighed. The wet-back expressed as grams of fluid was then calculated from the difference in weight of the filter pads. The results are the average of tests on five samples.

Results

| Sample | Fluid wet-back |
|--------|----------------|
| Sanitary towel of Example 1 | 0.22g |
| Comparison Sanitary Towel | 0.91g |

The comparison sanitary towel was a commercially available sanitary towel which had a construction similar to that of Example 1 except that the intermediate layer was a viscose rayon fleece (0.8g).

The results show that the sanitary towels of the invention which have an intermediate layer of embossed plastics net have a much reduced fluid wet-back when compared to that of prior art sanitary towels having an intermediate layer of viscose rayon fleece.

## CLAIMS

1.    An hygienic absorbent pad which comprises a liquid pervious cover layer, an absorbent layer and liquid pervious intermediate layer between the liquid pervious cover layer and the body facing surface of the absorbent layer characterised in that said intermediate layer comprises an embossed plastics net.

2.    An hygienic absorbent pad as claimed in Claim 1 in the form of a sanitary towel.

3.    A sanitary towel as claimed in Claim 2 in which the intermediate layer is a layer of embossed plastics net which covers the whole of the body facing surface of the absorbent layer.

4.    A sanitary towel as claimed in Claim 3 in which the intermediate layer of embossed plastics net forms part of a wrapper which surrounds the absorbent layer.

5.    A sanitary towel as claimed in any of Claims 2 to 4 in which the embossed plastics net has a thickness of 0.5mm to 1.2mm.

6.      A sanitary towel as claimed in any of Claims 2 to 5 in which the embossed plastics net comprises longitudinal ribs interconnected by fibrillated strands.

7.      A sanitary towel as claimed in any of Claims 2 to 6 in which the embossed plastics net comprises thicker junctures interconnected by thinner strands.

8.      A sanitary towel as claimed in any of Claims 2 to 7 in which the embossed plastics net has an alternating pattern of parallel linear raised and depressed areas.

9.      A sanitary towel as claimed in Claim 8 in which the embossed plastics net comprises longitudinal ribs and the linear raised and depressed areas in the net extend in a direction which is transverse to the longitudinal direction of the ribs.

10.     A sanitary towel as claimed in any of Claims 2 to 9 in which the embossed plastics net comprises polyolefine.

Fig.1

Fig.2

Fig.3